# EUROPEAN PATENT SPECIFICATION

(11) **EP 0 874 608 B1**
(45) Date of publication and mention of the grant of the patent: **16.04.2003**
(21) Application number: 96945679.7
(22) Date of filing: 31.12.1996
(51) Int. Cl.: A61F 5/56, A62B 7/00, A61M 16/06

(54) **DEVICE FOR IMPROVING BREATHING**
VORRICHTUNG ZUR VERBESSERUNG DES ATHMENS
DISPOSITIF D'AMELIORATION DE LA RESPIRATION

(30) Priority: 03.01.1996 US 582526; 31.01.1996 US 594904; 14.05.1996 US 645673
(43) Date of publication of application: 04.11.1998
(73) Proprietor: Thornton, W. Keith, Dallas, TX 75220 (US)
(72) Inventor: Thornton, W. Keith, Dallas, TX 75220 (US)
(74) Representative: Behrendt, Arne, Dipl.-Ing.
(86) International application number: US9620857
(87) International publication number: WO97025010

(56) References cited:
- WO-A-95/08969
- WO-A-95/33418
- DE-U- 29 506 512
- US-A- 2 424 533
- US-A- 4 169 473
- US-A- 4 470 413
- US-A- 4 669 459
- US-A- 5 365 945
- US-A- 5 409 017

## Description

### TECHNICAL FIELD OF THE INVENTION

This invention relates generally to medical devices, and more particularly to a device for improving breathing.

### BACKGROUND OF THE INVENTION

Many people experience breathing problems. These problems may result in difficulty sleeping, in snoring, or in more serious conditions such as sleep apnea.

One treatment for breathing disorders involves the use of devices inserted into a user's mouth for extending the user's lower jaw forward. These devices may open the user's breathing passageway more fully and thereby allow the user to breathe more easily through the nose and mouth. Although these devices may treat some breathing problems, these devices may not adequately treat more serious conditions such as sleep apnea. Furthermore, these devices often lack customizability and adjustability necessary to serve a variety of users and treatment requirements.

Another treatment for breathing disorders involves application of continuous positive air pressure ("CPAP") to the patient. The CPAP is delivered through a face mask, nose mask, or nasal inserts, and results in a fuller opening of the patient's breathing passageway. The CPAP may be increased for more serious conditions. Face masks for delivering CPAP are commonly secured to the patient's head with an adjustable or elastic strap. As the patient moves, the face mask often shifts and unseats from the patient's face, which may reduce the CPAP or awaken a sleeping patient.

US-A-5409017 discloses a mandible repostioning appliance having an upper bite block and a lower bite block interconnected by an adjustable mechanism including a posterior section connected to the rear portion of the upper bite block and an interior section connected to the front portion of the lower bite block and an adjustable interconnection between interior and posterior sections.

### SUMMARY OF THE INVENTION

The present invention addresses the disadvantages and problems associated with previous devices for improving breathing.

In particular, a device for improving the breathing of a user, comprising:
an upper arch adapted to receive at least some of the user's upper teeth;
a lower arch adapted to receive at least some of the user's lower teeth; and
a connector adjustably coupling the upper arch to the lower arch;
the connector allowing lateral motion of the lower arch relative to the upper arch,
characterised in that the connector is operable to be adjusted while the upper arch is coupled to the lower arch and while the device is inserted in the user's mouth to pull the lower arch forwardly to a fixed forward position relative to the upper arch.

In a further embodiment, the connector is removably coupled to the upper arch. In another embodiment, the connector includes a lip bumper. In another embodiment, the upper and lower arches are adapted to receive a deformable material in which molds of at least some of a user's upper and lower teeth, respectively, may be formed. In still another embodiment, the connector is operable to allow lateral motion of the lower arch relative to the upper arch, and to adjustably position the lower arch forwardly, relative to the upper arch, by exerting a tensile force upon the lower arch.

In a further embodiment, a device for improving breathing is provided which includes an upper arch adapted to receive at least some of a user's upper teeth, a lower arch adapted to receive at least some of a user's lower teeth, and a connector adjustably coupling the lower arch to the upper arch. The connector is operable to adjustably position the lower arch forwardly, relative to the upper arch, by exerting a tensile force upon the lower arch. The device also includes a support surface positioned forwardly, relative to the lower arch, and a tensile member having first and second ends. The first end of the tensile member is adjustably coupled to the connector, the second end of the tensile member is coupled to the lower arch, and the tensile member contacts the support surface along a contact region of the tensile member that is located between its first and second ends.

Yet another embodiment of the present invention provides a device for improving breathing which includes an upper arch adapted to receive at least some of a user's upper teeth, a connector operable to allow lateral motion of a user's lower jaw relative to the upper arch and to adjustably position the lower jaw forwardly relative to the upper arch, a face mask, a system for supplying gas to the face mask, and a connecting apparatus operable to adjustably couple the face mask to the upper arch. In a further embodiment, the connecting apparatus is operable to allow universal movement between the upper arch and the face mask.

In accordance with another embodiment of the present invention, a device for improving breathing includes an upper arch adapted to receive at least some of a user's upper teeth and a lower arch adapted to receive at least some of the user's lower teeth. A tensile member is coupled to the upper arch and the lower arch, and exerts a tensile force upon the lower arch. The device adjustably positions the lower arch forwardly, relative to the upper arch, using the tensile force. In another embodiment, the device includes an upper arch having a channel operable to receive the tensile member. In yet another embodiment, a plurality of tensile members adjustably position the lower arch forwardly, relative to the upper arch.

An important technical advantage of the present invention is that a face mask is anchored by customizable arches, thereby providing a more secure fit for the face mask on the user's face. The present invention provides a device for improving breathing that is readily customizable by the user or a clinical professional. Therefore, the device may be constructed so as to fit the user's mouth more securely and to more effectively treat the user's particular breathing disorder. Furthermore, because the device is customizable by the user as well as by a clinical professional, the device and associated treatment may be less expensive than would otherwise be possible. A lip bumper may also be provided which allows the user's lips to form a seal around a portion of the device that includes the upper arch, thereby allowing the CPAP system to work more effectively. The lip bumper also provides additional comfort by reducing fatigue that may result from a user's tendency to form the user's lips more tightly around an object whose cross-section is smaller than the opening formed by the lips when they are in a relaxed state.

Another important technical advantage of the present invention is the fact that it allows a user's lower jaw to be adjustably positioned forwardly relative to the user, while permitting the jaw to move laterally. Therefore, the user's comfort may be increased without reducing the effectiveness of the device. In particular embodiments, forward adjustment is done from a point forward relative to the jaw, reducing the upward vertical load experienced by the lower arch and the likelihood that the lower arch will be pulled off of the lower teeth when the user's lower jaw is moved. In some embodiments, forward adjustment of the lower jaw is accomplished using one or more tensile members that may be adjusted independently or together. As a result, the device of the present invention provides increased adaptability, remaining more comfortable for the user, while more effectively treating serious breathing disorders such as sleep apnea.

### BRIEF DESCRIPTION OF THE DRAWINGS

For a more complete understanding of the present invention and its advantages, reference is now made to the following descriptions taken in conjunction with the accompanying drawings, in which like reference numbers indicate like features and wherein:
FIGURE 1a illustrates an isometric view of a device for improving breathing constructed according to the teachings of the present invention;
FIGURE 1b illustrates an alternative embodiment of the present invention adapted for use in connection with alternative upper and lower arches;
FIGURES 2a-2d illustrate bottom isometric, side, front, and bottom views of the device illustrated in FIGURE 1;
FIGURES 3a and 3b illustrate side and front views of an alternate embodiment of the present invention adapted for use in connection with a slider;
FIGURES 4a-4c illustrate exploded bottom, front, and side views of a hooking element constructed according to the teachings of the present invention;
FIGURES 5a-5d illustrate exploded side views of alternative embodiments of the present invention adapted for use in connection with alternative hooking elements and a flexible member;
FIGURES 6a-6c illustrate exploded side views of alternative embodiments of the present invention adapted for use in connection with a tensile member;
FIGURE 7 illustrates an exploded side view of an alternative embodiment of the present invention adapted for use in connection with a tongue stud;
FIGURE 8 illustrates another device for improving breathing;
FIGURES 9a-9c illustrate exploded views of adjustors;
FIGURE 10 illustrates an alternative device for improving breathing;
FIGURE 11 illustrates an exploded view of an alternative adjuster; and
FIGURE 12 illustrates another alternative device for improving breathing.

### DETAILED DESCRIPTION OF THE INVENTION

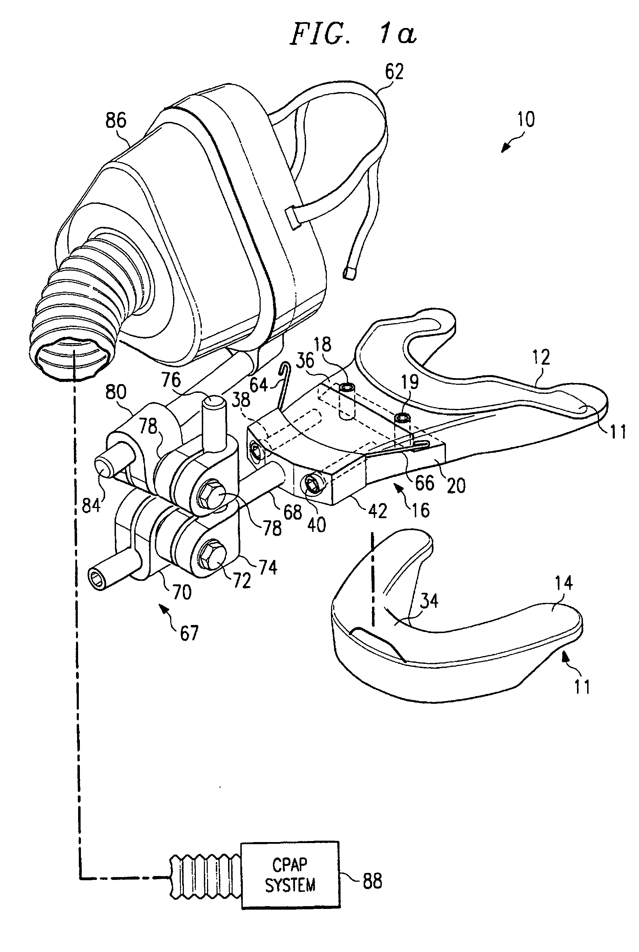

FIGURE 1a illustrates a device for improving breathing constructed according to the teachings of the present invention. Device 10 is provided with an upper arch 12 and a lower arch 14. Upper arch 12 is adapted to receive at least some of a user's upper teeth, and lower arch 14 is adapted to receive at least some of the user's lower teeth. When device 10 is in use, upper arch 12 and lower arch 14 are inserted into the user's mouth.

Upper arch 12 and lower arch 14 are adapted to receive a deformable material 11 in which molds of at least some of the user's upper and lower teeth, respectively, may be formed. Deformable material 11 may be the ethylene-vinyl acetate copolymer resin sold under the name ELVAX ®, or another suitable deformable material that can be used to form molds of a user's teeth. In one embodiment, deformable material 11 is heated to a temperature of about 65.56°C (one hundred and fifty degrees Fahrenheit), using a microwave oven or hot water, for example, so as to place deformable material 11 in its deformable state. Upper arch 12 and lower arch 14 are then inserted into the user's mouth, either separately or together, and the user bites down to deform deformable material 11 into the shape of the user's teeth. Upper arch 12 and lower arch 14 are then removed and allowed to cool and harden. The present invention is therefore readily customizable by the user.

Alternatively, as shown in FIGURE 1b, upper arch 12 and lower arch 14 may themselves be formed from a deformable material suitable for dental uses, such as methyl methacrylate or a polycarbonate resin thermoplastic such as that sold under the name LEXAN ®. Such materials are known to those familiar with dental mouthpieces, and other materials may be used without departing from the intended scope of the present invention. Thus, the present invention is also readily customizable by a clinical professional.

Various securing clasps can be used to more fully secure upper arch 12 and lower arch 14 to the user's teeth. Illustrative embodiments include C-clasps 13, ball clasps 15, and U-clasps 17. One or more of securing clasps 13, 15, and 17 may be included on upper arch 12 and lower arch 14.

Upper arch 12 is coupled to the remainder of device 10 using connector 16. The body 20 of connector 16 is removably coupled to upper arch 12 using screws 18 and 19. Other fastening agents may be employed without departing from the intended scope of the present invention. Although upper arch 12 is removably coupled to body 20 of connector 16, the present invention also contemplates an integral piece of material that forms both body 20 and upper arch 12 without the need for fastening agents.

An important advantage of the present invention is that body 20 of connector 16 is removable from upper arch 12. This allows a mold of the user's upper teeth to be formed before device 10 is assembled and secured to the user. Similarly, as discussed more fully below, lower arch 14 is removably coupled to connector 16. Lower arch 14 may therefore include a mold of the user's lower teeth that is formed before device 10 is assembled and secured to the user. Because upper arch 12 and lower arch 14 are removably coupled to connector 16, and are therefore removably coupled to the remainder of device 10, the present invention may be customized by either the user or by a clinical professional without involving the remainder of device 10.

Connector 16 may provide a lip bumper 36 that allows the user's lips to form a seal around body 20. Lip bumper 36 reduces fatigue that may result from a user's tendency to form his or her lips tightly around an object whose cross-section is smaller than the opening formed by the user's lips when they are in a relaxed state. Lip bumper 36 also provides a more effective seal between the user's lips and body 20 to increase the effectiveness of the CPAP system discussed more fully below. Lip bumper 36 may be integral to or separate from connector 16. Alternatively, lip bumper 36 may be provided by upper arch 12 rather than by connector 16 when, for example, necessitated by the location of the coupling between upper arch 12 and connector 16. Lip bumper 36 may be integral to or separate from upper arch 12 in such a case.

Body 20 is coupled to face plate 42 using screws 38 and 40, or other suitable fastening agents. It should be understood that the present invention contemplates, as an alternative embodiment, an integral piece of material that forms both body 20 and face plate 42 without the need for fastening agents.

Device 10 may be adapted for use in connection with a face mask 86 and a continuous positive air pressure ("CPAP") system 88 for supplying a gas to face mask 86. Face mask 86 may be configured so as to allow gas to be supplied by CPAP system 88 at constant positive air pressure, or at a positive air pressure adjusted accordingly for the particular user or breathing disorder being treated. In general, increasing the air pressure delivered by CPAP system 88 increases the opening of the breathing passageway. CPAP system 88 is shown as an example only. Other systems for delivering any breathable gas, such as air, oxygen, or gases used in anaesthesia, at constant or varying pressures may also be used. Face mask 86 should be configured so as to allow the gas exhaled by the patient to be exhausted from face mask 86.

According to one embodiment of the present invention, an adjustable strap 62 may be included with device 10. Adjustable strap 62 is operable to engage strap hooks 64 and 66, which are coupled to face plate 42 of connector 16. Adjustable strap 62 operates to secure device 10 to the user's head, and consequently allows the user's lower jaw to be extended forward with less stress placed on the user's upper jaw and less risk of injury to the user's teeth. Adjustable strap 62 also increases the effectiveness of CPAP system 88 in delivering gas to face mask 86. Adjustable strap 62 need not engage both or either of strap hooks 64 and 66, and may instead attach only to face mask 86. Conversely, adjustable strap 62 may be attached only to a portion of connector 16, such as strap hooks 64 and 66, but not to face mask 86. Other methods of coupling adjustable strap 62 to device 10 may also be used.

In another embodiment of the present invention, face mask 86 is adjustably coupled to connector 16 and therefore to upper arch 12 using connecting apparatus 67. Connecting apparatus 67 includes a hollow post 68 that extends forward from face plate 42. Hollow post 68 meets and engages swivel collar 70, forming an adjustable collar joint through which hollow post 68 may slide and rotate. Fastener 72 couples swivel collars 70 and 74 at a desired angle relative to one another. Connecting post 76 extends at one end through swivel collar 74, forming a second adjustable collar joint through which connecting post 76 may slide and rotate. When fastener 72 is tightened, the two collar joints formed by collars 70 and 74 with hollow post 68 and connecting post 76, respectively, are tightened.

Connecting post 76 may extend at the other end through collar 78, forming a third adjustable collar joint through which connecting post 76 may slide and rotate. Fastener 80 couples swivel collars 78 and 82 at a desired angle relative to one another. Post 84 may extend at its distal end through swivel collar 82, forming a fourth collar joint through which post 84 may slide and rotate. When fastener 80 is tightened, the collar joints formed by swivel collars 78 and 82 with connecting post 76 and post 84, respectively, are tightened.

Post 84 may be secured to face mask 86 at its proximal end, completing the coupling of connector 16 to face mask 86. The present invention may thereby provide universal adjustability, allowing the portion of device 10 that is coupled to the user's mouth to be adjustably positioned forward, vertically, laterally, angularly, or rotationally with respect to face mask 86, providing an important technical advantage. It should be understood that other methods of providing universal or lesser adjustability may be employed with device 10 without departing from the intended scope of the present invention.

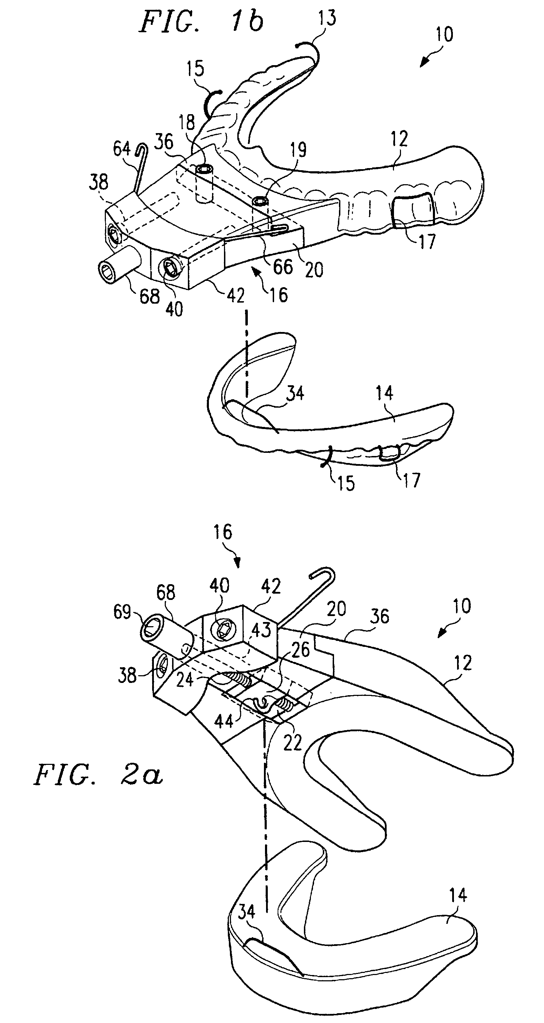

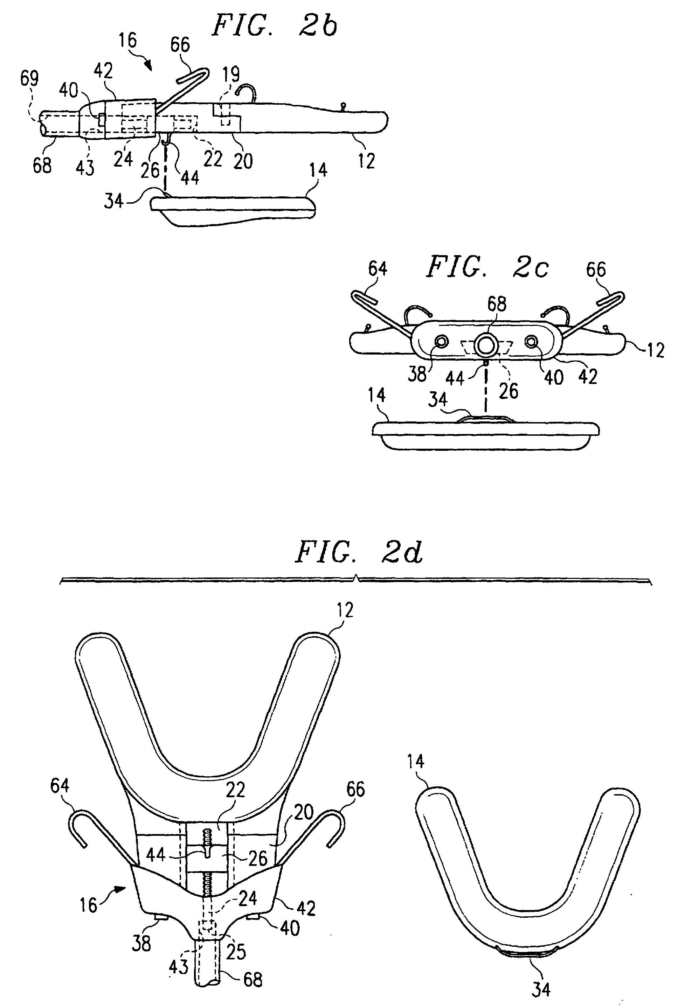

FIGURES 2a-2d illustrate a body 20 of connector 16 having a channel 22. Hooking element 26 is adapted to fit in channel 22 and to slide along the length of channel 22. Horizontal screw 24 extends from the front of body 20 rearward into channel 22 to engage hooking element 26. Screw head 25 of horizontal screw 24 may be accessed using a horizontal hole 43 formed in face plate 42.

A screwdriver or other suitable adjustment device may be inserted into horizontal hole 43 in face plate 42 to engage screw head 25. The hollow portion 69 of hollow post 68, which is coupled to connector 16 through face plate 42, provides access to horizontal hole 43 and to screw head 25. Horizontal screw 24 may extend forward through face plate 42 and into hollow portion 69, may extend only into horizontal hole 43 and not into hollow portion 69, or rest only within body 20 and not extend into horizontal hole 43.

In one embodiment, at least a portion of the side walls of channel 22 are formed diagonally so as to guide the travel of hooking element 26 forward or rearward within channel 22 when horizontal screw 24 is turned, while not allowing hooking element 26 to displace vertically. Hooking element 26 slides forward or rearward within channel 22 in response to horizontal screw 24 being turned in the appropriate direction. The present invention contemplates side walls of channel 22 that are formed in any manner suitable to allow hooking element 26 to travel forward or rearward within channel 22, while not allowing hooking element 26 to displace vertically.

Lower arch 14 includes a hooking clasp 34, which extends laterally across the midline of lower arch 14 and is operable to engage hook 44. While engaged with hook 44, lower arch 14 may move laterally in response to lateral motions of the user's jaw. Hook 44 may be integral to or separate from hooking element 26. Hooking clasp 34 is shown with a substantially circular cross-section that allows coupling between hooking clasp 34 and hook 44, but other cross-sections of hooking clasp 34 and manners of coupling hooking element 26 and hooking clasp 34 may be used without departing from the intended scope of the present invention.

Due to the relative positions of hook 44 and hooking clasp 34, when hook 44 has engaged hooking clasp 34, lower arch 14 and thus the user's lower jaw may be extended forward, relative to upper arch 12. When horizontal screw 24 is turned in the appropriate direction, lower arch 14 and thus the user's lower jaw may be adjusted forward or rearward so as to be extended forward to the extent desired, relative to upper arch 12. The amount of forward extension may be increased or decreased as necessary to properly size device 10 to the user and to effectively treat the breathing disorder involved. In another embodiment, hooking element 26 is allowed to move freely through channel 22 unless a vertical screw or other suitable member is adjusted to contact hooking element 26 within channel 22 and thereby press hooking element 26 against the inner surface of channel 22 with a force sufficient to secure hooking element 26 in the desired position. Hooking element 26 may be further adjusted by reducing or releasing the force pressing hooking element 26 against channel 22, moving hooking element 26 to the desired position, and then reapplying the force to hooking element 26. The present invention contemplates alternate embodiments wherein the lower jaw is adjusted using a motor or hydraulics. Such a motor or hydraulics may be used in connection with horizontal screw 24, but need not be used in connection with horizontal screw 24 to be within the intended scope of the present invention.

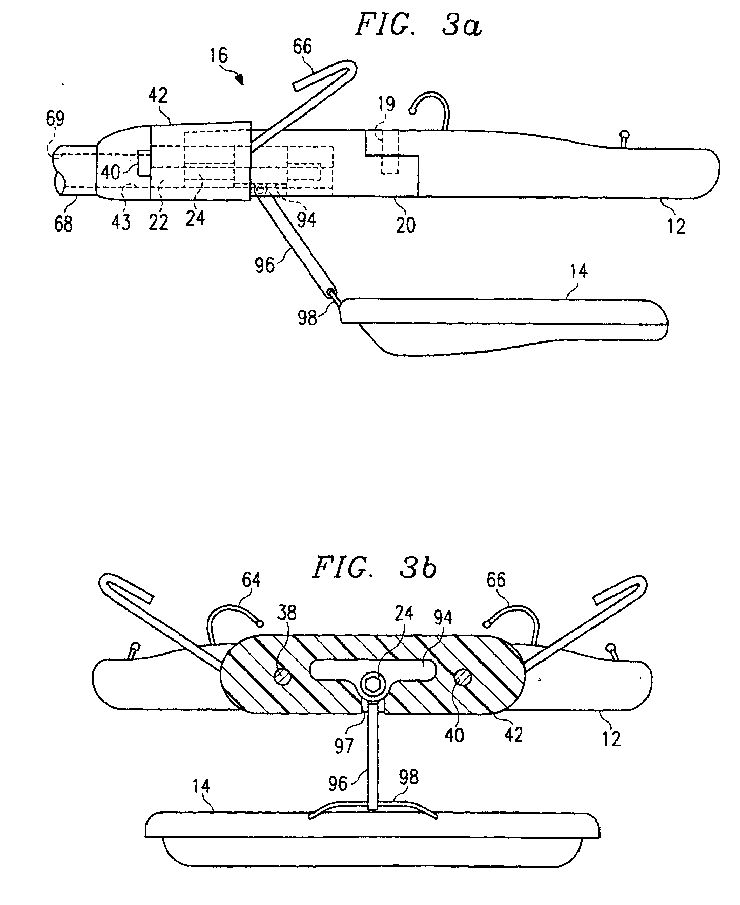

FIGURES 3a and 3b illustrate side and front views of an alternate embodiment of the present invention adapted for use in connection with a slider 94. Slider 94 is adapted to fit in channel 22 and to slide along the length of channel 22. Horizontal screw 24 extends from the front of body 20 rearward into channel 22 to engage slider 94. Screw head 25 of horizontal screw 24 may be accessed and adjusted as described above in connection with FIGURES 2a-2d.

In one embodiment, at least a portion of the side walls of channel 22 are formed to cooperate with the shape of slider 94 so as to guide slider 94 forward or rearward within channel 22 when horizontal screw 24 is turned, while not allowing slider 94 to displace vertically. Slider 94 slides forward or rearward within channel 22 in response to horizontal screw 24 being turned in the appropriate direction. The present invention contemplates side walls of channel 22 that are formed in any manner suitable to allow slider 94 to travel forward or rearward within channel 22, while not allowing slider 94 to displace vertically.

Connecting arm 96 is coupled to slider 94 and extends generally downward from slider 94, through the slot 97 of channel 22, to a region generally below upper arch 12. Lower arch 14 includes a lateral bar 98 that is operable to engage connecting arm 96 at its distal end. While engaged with connecting arm 96, lower arch 14 may move laterally in response to lateral motions of the user's lower jaw. Connecting arm 96 may be integral to or separate from slider 94. Connecting arm 96 may be of variable length. Lateral bar 98 is shown with a substantially circular cross-section that allows coupling between connecting arm 96 and lateral bar 98; but other cross-sections of lateral bar 98 and manners of coupling connecting arm 96 and lateral bar 98 may be used without departing from the intended scope of the present invention.

Due to the relative positions of connecting arm 96 and lateral bar 98, when connecting arm 96 has engaged lateral bar 98, lower arch 14 and thus the user's lower jaw may be extended forward, relative to upper arch 12. The user's jaw may be adjusted in the same manner as described above in connection with FIGURES 2a-2d.

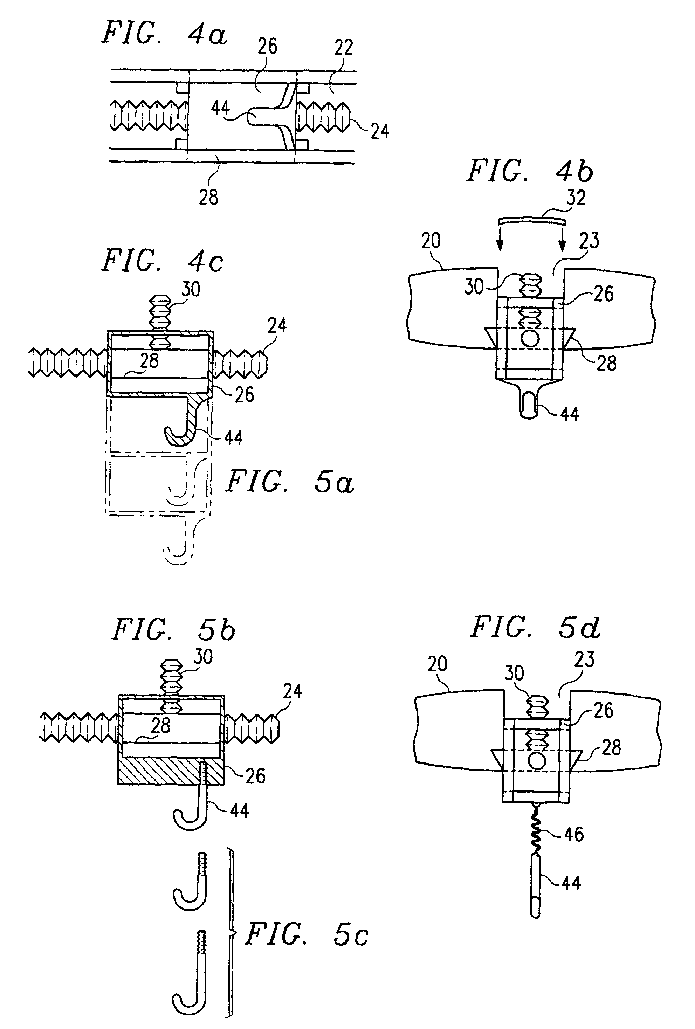

FIGURES 4a-4c illustrate exploded bottom, front, and side views of alternative embodiments of the present invention that include an alternative hooking element 26 constructed according to the teachings of the present invention. Hooking element 26 is vertically as well as forwardly adjustable, allowing it to be raised or lowered as necessary to customize device 10 for the particular user and breathing disorder being treated.

As shown in FIGURES 4b and 4c, vertical screw 30 extends downward from the exposed upper portion 23 of channel 22, through a threaded hole in the top of hooking element 26, until vertical screw 30 contacts flange 28, which is separate from hooking element 26. Vertical screw 30 may travel forward or rearward within exposed upper portion 23 of channel 22 when hooking element 26 and flange 28 slide within channel 22 in response to horizontal screw 24 being adjusted. Being exposed from the top, vertical screw 30 is operable to adjust the vertical displacement of hooking element 26 from flange 28. In one embodiment, vertical screw 30 is a set screw. Because flange 28 is prevented from moving vertically due to the shape of the side walls of channel 22, turning vertical screw 30 causes hooking element 26 to displace vertically, relative to upper arch 12. A removable cap 32 may be included in order to cover exposed upper portion 23 of channel 22 when vertical screw 30 is not being adjusted.

Due to the complimentary shapes of channel 22, hooking element 26, flange 28, and vertical screw 30, hooking element 26 is freely adjustable forwardly, rearwardly, and vertically. The ability of device 10 to be freely adjusted in this manner is a significant technical advantage over prior art devices, adding to the ease, effectiveness, and comfort of device 10 in improving breathing disorders. The present invention contemplates alternate embodiments wherein hooking element 26 is adjusted using a motor or hydraulics.

FIGURES 5a-5d illustrate exploded side views of alternative embodiments of the present invention. As shown in FIGURE 5a, the vertical length of hooking element 26 is variable, such that the vertical distance between lower arch 14 and upper arch 12 may be increased. Hooking element 26 remains freely adjustable forwardly, rearwardly, and vertically. As shown in FIGURE 5b, hook 44 may be fixedly attached or removably attached to hooking element 26. Hook 44 may be glued or otherwise affixed to hooking element 26, insertionally glued or force fitted into a hole drilled into hooking element 26, screwed into hooking element 26, or attached in any other manner to hooking element 26. As shown in FIGURE 5c, the vertical length of hook 44 is also variable, such that an appropriate combination of hook 44 and hooking element 26 may be made in order to achieve the desired vertical displacement of lower arch 14, relative to upper arch 12. The fact that every such combination is not shown herein does not in any way limit the intended scope of the present invention.

FIGURE 5d illustrates an alternative embodiment of hook 44 that includes a flexible member 46, such as a spring or rubberband, disposed between connector 16 and hook 44. When hook 44 has engaged hooking clasp 34 of lower arch 14 so as to extend the user's lower jaw forward, the jaw may still enjoy some freedom of movement due to flexible member 46. Not only does this configuration enable the user to be more comfortable when using device 10, flexible member 46 reduces any risk that the user's teeth may be injured as a result of quick and forceful movement of the user's lower jaw. Flexible member 46 may be incorporated into any of the embodiments described above or into any combination thereof, whether or not shown or described. In another embodiment, flexible member 46 is fixedly or removably attached to connector 16 at one end and fixedly or removably attached to lower arch 14 at the other end, coupling connector 16 to lower arch 12 directly. Other mechanisms designed to dampen the force transferred to and between upper arch 12 and lower arch 14 may be used without departing from the intended scope of the present invention.

FIGURES 6a-6c illustrate side views of other embodiments of the present invention adapted for use in connection with a tensile member 48 under tension. According to one embodiment, tensile member 48 may comprise a monofilament line. According to alternate embodiments, tensile member 48 may be constructed in any shape and from any material suitable to extend the user's lower jaw forward when tensile member 48 is placed under tension, without departing from the intended scope of the present invention.

As shown in FIGURES 6a and 6b, tensile member 48 is secured to tension screw 50 at the generally top and forward surface of face plate 42 of connector 16. Tension screw 50 is operable to displace tensile member 48 in response to adjustment of tension screw 50. From its attachment at tension screw 50, tensile member 48 extends generally downward through hole 52, contacts support surface 54 as it leaves hole 52 at the generally bottom surface of face plate 42, and extends to attachment point 55 on lower arch 14. Attachment point 55 may be at any suitable location on lower arch 14. When tension screw 50 is adjusted, lower arch 14 is pulled in the direction of tensile member 48. The user's lower jaw is therefore extended forward, relative to upper arch 12. The present invention contemplates other suitable configurations of tension screw 50, hole 52, and support surface 54, so long as lower arch 14 is pulled generally forward in the direction of tensile member 48 when tension screw 50 is adjusted. In another embodiment, tensile member 48 is allowed to move freely through hole 52 unless a screw or other suitable member is adjusted to contact tensile member 48 within hole 52 and thereby press tensile member 48 against the inner surface of hole 52 with a force sufficient to secure tensile member 48 in the desired position. Tensile member 48 may be further adjusted by reducing or releasing the force pressing tensile member 48 against the inner surface of hole 52, moving tensile member 48 to the desired position, and then reapplying the force to tensile member 48. The present invention contemplates alternate embodiments wherein lower arch 14 is adjusted using a motor or hydraulics in connection with tensile member 48.

An important technical advantage of the present invention is that it allows the user's lower jaw to be adjustably positioned forwardly in response to tension directed from a point forward, relative to the user's lower jaw. The upward vertical component of the tension on lower arch 14 is decreased the more nearly horizontal the direction of tension placed on lower arch 14 using tensile member 48. As the upward vertical component of the tension decreases, so does the possibility that lower arch 14 will be pulled off the user's lower teeth when the mouth is opened or the lower jaw is moved. It is therefore desirable to direct the tension on lower arch 14 from a point forward of lower arch 14 and below attachment point 55 of tensile member 48 or as nearly horizontal with attachment point 55 as possible.

Hole 52 may be formed so as to cause tensile member 48 to contact the inner surface of hole 52 at support surface 54 before extending to attachment point 55. Tensile member 48 is thereby redirected such that the angle of its attachment to lower arch 14, relative to the horizontal, is more acute than it would be if tensile member 48 extended in a straight line between tension screw 50 and attachment point 55. The vertical component of the tension on lower arch 14 is thereby reduced. It should be understood that the positions and angles of hole 52 and support surface 54 may be varied in order to adjust the angle at which tensile member 48 attaches to lower arch 14, thereby adjusting the vertical component of the tension on lower arch 14.

As explained above, reducing the upward vertical component of the tension on lower arch 14 reduces the possibility that lower arch 14 will be pulled off the user's lower teeth when the mouth is opened or the lower jaw is moved. As shown in FIGURE 6b, support arm surface 57 may be located near the distal end of support arm 56, which extends generally downward from connector 16 to a point generally forward of lower arch 14. Tensile member 48 contacts and is redirected by support arm surface 57. Tensile member 48 may also contact and be redirected by support surface 54. Support arm surface 57 is positioned vertically so as to allow tensile member 48 to exert a tensile force on lower arch 14 in a generally horizontal direction when tension screw 50 is appropriately adjusted. As discussed above, this provides an important technical advantage of the present invention.

Support arm 56 may be integral to or separate from connector 16. Support arm 56 may be fixedly coupled to connector 16, or may be coupled to connector 16 in a manner that allows support arm 56 to be extended or retracted by adjusting extension screw 59 in the appropriate direction. Variations in the manner of attachment of support arm 56 to device 10 and in the adjustability of support arm 56 are within the intended scope of the present invention. Using any such configuration to properly position support arm surface 57 vertically, through the positioning of support arm 56, the user or clinical professional can reduce or eliminate the upward vertical component of the tension on lower arch 14.

In one embodiment, support arm surface 57 is provided by a hole in the distal end of support arm 56. In another embodiment, support arm 56 has a bifurcated distal end to constrain tensile member 48 laterally as it contacts and travels over support arm surface 57. Alternatively, a pulley 58 mounted on the distal end of support arm 56 provides support arm surface 57. Other configurations suitable to provide support arm surface 57 may be used without departing from the intended scope of the present invention.

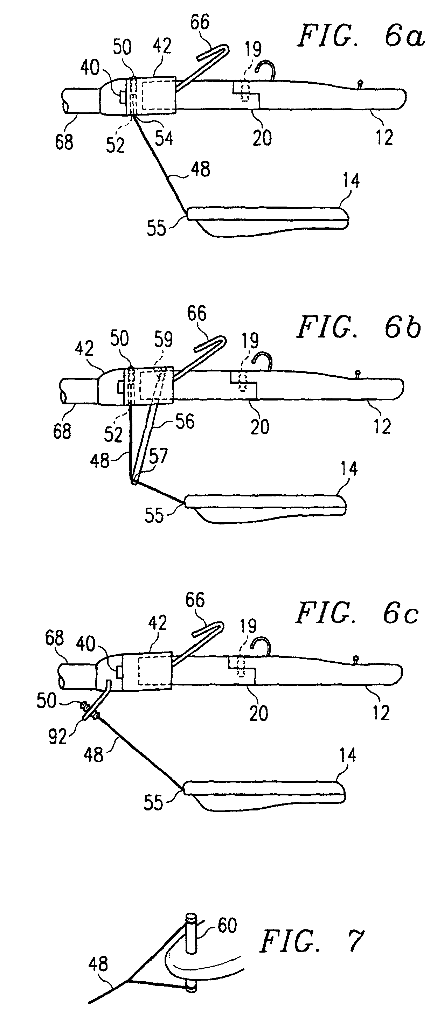

FIGURE 6c illustrates an embodiment of the present invention wherein the point at which tensile member 48 is coupled to connector 16 is generally below face plate 42. A mount 92 projects generally downward from connector 16 and adjustably couples tension screw 50, which is coupled to tensile member 48, to connector 16. As before, tensile member 48 is secured to lower arch 14 at attachment point 55, allowing lower arch 14 to be adjusted forwardly when tension screw 50 is turned. The location and vertical length of mount 92 may be varied as necessary to properly size device 10 to the user and to effectively treat the breathing disorder involved. The upward vertical component of the tension placed on lower arch 14 may be reduced or eliminated by configuring device 10 such that attachment point 55 is nearly level with or above the coupling of tensile member 48 to mount 92.

FIGURE 7 illustrates an exploded side view of an alternative embodiment of the present invention adapted for use in connection with a tongue stud 60. Tongue stud 60 is made to pierce the user's tongue and is then secured to the remainder of device 10. Tongue stud 60 may be used in connection with any or all of the embodiments of the present invention described above to replace or combine with the functions of lower arch 14. For example, tongue stud 60 may be coupled to tensile member 48 in any of the embodiments illustrated in FIGURES 6a-6c. When used in connection with tensile member 48, tongue stud 60 is operable to extend the user's tongue forward when tension is placed on tensile member 48, thereby improving the breathing of the user. Although tongue stud 60 has been described most thoroughly in connection with tensile member 48, it should be understood that the present invention contemplates using tongue stud 60 in connection with any or all of the embodiments described above. For example, tongue stud 60 may be coupled to hook 44, hooking element 26, connecting arm 96, slider 94, or any other part of connector 16 in any or all of the embodiments discussed above. The present invention contemplates alternative embodiments wherein tongue stud 60 is positioned using a motor or hydraulics.

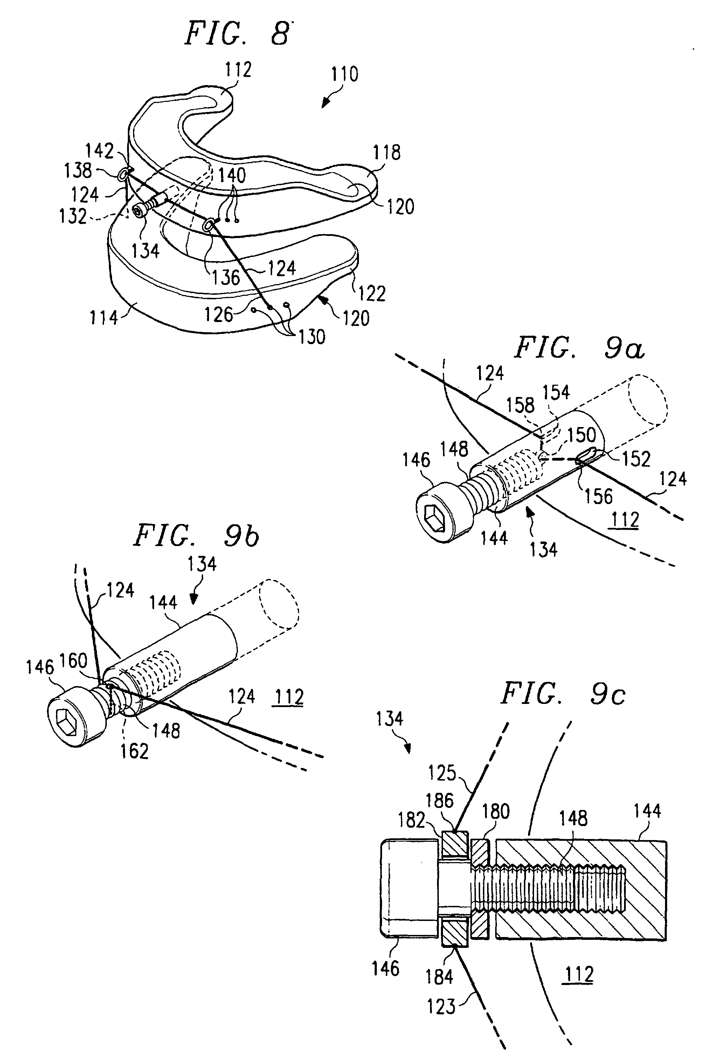

FIGURE 8 illustrates a device for improving breathing, indicated generally at 110, that includes an upper arch 112 adapted to receive at least some of a user's upper teeth and a lower arch 114 adapted to receive at least some of the user's lower teeth. When device 110 is in use, upper arch 112 and lower arch 114 are inserted into the user's mouth. Upper and lower arches 112 and 114 include trays 118 and 122, respectively. Trays 118 and 122 may be formed from any material suitable for dental uses, for example, methyl methacrylate or a polycarbonate resin thermoplastic such as that sold under the Registered Trademark Lexan. Such materials are well-known to those familiar with dental devices, and other suitable materials may be used to form trays 118 and 122 without departing from the intended scope of the present invention.

Trays 118 and 122 are adapted to receive a deformable material 120 in which molds of at least some of the user's upper and lower teeth, respectively, may be formed. Deformable material 120 may be formed from any suitable material, for example, a polyvinyl acetate such as the ethylene-vinyl acetate copolymer resin sold under the Registered Trademark Elvax. Any deformable material 120 suitable to form a mold of at least some of the user's upper and lower teeth may be used. As discussed more fully below, by forming a mold of the particular user's teeth, device 110 may be customized to fit the user.

In one embodiment, deformable material 120 is heated to a temperature of approximately 65.56°C (one hundred and fifty degrees Fahrenheit), using a microwave oven or hot water, for example, so as to place deformable material 120 in its deformable state. Upper arch 112, lower arch 114, or both upper arch 112 and lower arch 114 are then inserted into the user's mouth, either separately or together. The user bites down or otherwise presses at least some of the user's teeth into deformable material 120 in order to form a mold of the user's teeth. Deformable material 120 is then allowed to cool and harden or otherwise take a more permanent shape. These steps may be repeated as many times as necessary or desired in order to form a mold of at least some of the user's upper and lower teeth using deformable material 120.

Alternatively, upper arch 112 and lower arch 114 may themselves be formed from a deformable material suitable for dental uses, for example, methyl methacrylate or a polycarbonate resin thermoplastic such as that sold under the Registered Trademark Lexan. Upper arch 112 and lower arch 114 may be customized to fit the user's teeth in the user's home, in the office of a clinical professional, or in any other suitable location. Upper arch 112 and lower arch 114 may be customized to fit the user's teeth before, during, or after the remainder of device 110 is assembled or otherwise coupled to upper arch 112 and lower arch 114. The present invention contemplates using any combination of materials and techniques suitable to form a mold of at least some of the user's upper and lower teeth.

Whether upper arch 112 and lower arch 114 are formed from a deformable material or are constructed so as to include deformable material 120, properly fitting device 110 to the user's teeth increases the safety and effectiveness of device 110 in treating the particular breathing disorder for which device 110 was constructed. The present invention contemplates using the relining methods discussed in copending application Serial No. 08/621,133, filed March 21, 1996, to further improve the fit between upper and lower arches 112 and 114, respectively, and the user's teeth.

Furthermore, various securing clasps, for example, C-clasps, ball clasps, and U-Clasps, may be coupled to upper arch 112 and lower arch 114 to more fully secure upper arch 112 and lower arch 114 to the user's teeth when device 110 is in use.

Using tensile member 124, lower arch 114 may be adjustably extended forward, relative to upper arch 112. Tensile member 124 has first and second ends 126 and 128, respectively, that are coupled to lower arch 114 using attachments sites 130 and 132, respectively. The length of tensile member 124 is variable and is chosen to suit a particular user or application of device 110. In one embodiment, the locations of attachment sites 130 and 132 on lower arch 114 are also variable. The particular attachment sites 130 and 132 that couple first and second ends 126 and 128, respectively, to lower arch 114 are chosen from among a plurality of available attachment sites 130 and 132. Attachment sites 130 and 132 may include, without limitation, hooks for tying first and second ends 126 and 128 to lower arch 114, holes for force-fitting or gluing first and second ends 126 and 128 into lower arch 114, or any other mechanism for coupling first and second ends 126 and 128, respectively, to lower arch 114.

Adjustor 134 is coupled to upper arch 112 and is coupled to tensile member 124 between first end 26 and second end 128 of tensile member 124. Adjustor 134 may be integral to or separate from upper arch 112. Adjustor 134 may be formed from the same material as upper arch 112 or from a different material than upper arch 112. In one embodiment, adjustor 134 includes a tubular housing that is fixedly attached to upper arch 112. As discussed more fully below in connection with FIGURES 9a-9c, adjustor 134 is used to adjust the position of tensile member 124.

Tensile member 124 can be made to exert a tensile force upon lower arch 114 when device 110 is properly inserted into the user's mouth. The tensile force exerted by tensile member 124 upon lower arch 114 increases when adjustor 134 is appropriately adjusted, thereby pulling lower arch 114 forward, relative to upper arch 112. Similarly, the tensile force exerted by tensile member 124 upon lower arch 114 decreases when adjustor 134 is appropriately adjusted, thereby allowing lower arch 114 to retract, relative to upper arch 112. In this manner, lower arch 114 may be adjustably positioned forwardly, relative to upper arch 112, using adjustor 134. The present invention contemplates any adjustor 134 suitable for adjustably positioning lower arch 114, relative to upper arch 112. The present invention further contemplates two or more tensile members to replace or combine with the functions of tensile member 124.

Support loops 136 and 138 are coupled to upper arch 112 using attachment sites 140 and 142, respectively. In one embodiment, the locations of attachment sites 40 and 142 on upper arch 112 are variable. The particular attachment sites 140 and 142 used to couple support loops 136 and 138, respectively, to upper arch 112 are chosen from among a plurality of available attachment sites 140 and 142. Tensile member 124 contacts and is redirected by support loop 136 along a first loop contact region of tensile member 124 between adjustor 134 and first end 126 of tensile member 124. Similarly, tensile member 124 contacts and is redirected by support loop 138 along a second loop contact region of tensile member 124 between adjustor 134 and second end 128 of tensile member 124.

In combination, support loops 136 and 138 and attachment sites 130 and 132, respectively, define the angle at which tensile member 124 couples to lower arch 114. Therefore, support loops 136 and 138 and attachment sites 130 and 132, respectively, also define the angle from which tensile member 124 exerts a tensile force upon lower arch 114. By varying the relative positions and constructions of support loops 136 and 138 and attachment sites 130 and 132, respectively, the forward and vertical components of the tensile force may be defined according to the particular user or application of device 110. For example, it may be desirable to increase the forward component of the tensile force, relative to the vertical component, such that lower arch 114 is not pulled off of the user's lower teeth when the mouth is opened or the lower jaw is otherwise moved. In addition, due to the configuration of lower arch 114, tensile member 124, and support loops 136 and 138, lower arch 114 may move laterally in response to lateral movements of the lower jaw.

The present invention contemplates any suitable support surfaces to replace or combine with the functions of support loops 136 and 138. The present invention contemplates support loops 136 and 138 coupled to lower arch 114 at variable attachment sites on lower arch 114 instead of, or in addition to, support loops 136 and 138 coupled to upper arch 112. Using any suitable combination of tensile member 124, adjustor 134, support loops 136 and 138, and attachment sites 130 and 132, and 140 and 142, lower arch 114 may be adjusted forwardly, relative to upper arch 112, by exerting a tensile force upon lower arch 114.

FIGURES 9a-9c illustrate exploded views of adjusters for use in connection with device 110. As shown in FIGURE 9a, adjustor 134 includes a housing 144 coupled to upper arch 112 and an adjustment element 146. Housing 144 may be integral to or separate from upper arch 112. Housing 144 may be formed from the same materials as upper arch 112 or from different materials than upper arch 112. Housing 144 may be entirely exterior to upper arch 112 or may lie partially within upper arch 112. In one embodiment, housing 144 is fixedly attached to upper arch 112 by force-fitting, gluing, or otherwise inserting a portion of housing 144 into a hole in the outward surface of upper arch 112. The present invention contemplates forming housing 144 from any suitable material and coupling housing 144 to upper arch 112 in any suitable manner.

In one embodiment, housing 144 is tubular and is threaded along at least a portion of its interior surface. Adjustment element 146 includes a completely or partially threaded shaft 148 that mates with the threads on the interior surface of housing 144. Adjustment element 146 is coupled to tensile member 124 using a hooking element 150 located near the proximal end of adjustment element 146. Tensile member 124 passes through hooking element 50 along a coupling region of tensile member 124. Tensile member 124 is displaced forwardly, relative to upper arch 112, when hooking element 150 is displaced forwardly. The present invention contemplates coupling two or more tensile members to hooking element 150 in order to replace or combine with the functions of tensile member 124. As discussed more fully below, hooking element 150 maintains its rotational orientation, relative to housing 144, when adjustment element 146 is displaced forwardly.

In one embodiment, housing 144 includes slots 152 and 154. Slots 152 and 154 are located between hooking element 150 and support loops 136 and 138, respectively. Tensile member 124 passes through slots 152 and 154. When adjustment element 146 is displaced forwardly, relative to upper arch 112, tensile member 124 is pulled forwardly by hooking element 150. Tensile member 124 contacts and is redirected by support surfaces 156 and 158 along first and second surface contact regions of tensile member 124, respectively. When adjustment element 146 has been displaced forwardly to a sufficient extent, relative to upper arch 112, the friction forces generated at hocking element 150, support surfaces 156 and 158, and support loops 136 and 138 impede or prevent tensile member 124 from moving laterally through hooking element 150. Therefore, lower arch 114 is displaced equally by first end 126 and second end 128 of tensile member 124 when adjustment element 146 is displaced.

In one embodiment, adjustment element 146 is a cap screw that is displaced, relative to upper arch 112, by rotating the cap screw in the appropriate direction. Hooking element 150 is constructed and coupled to adjustment element 146 so as to maintain the rotational orientation of hooking element 150, relative to housing 144, when adjustment element 146 rotates. As a result, the coupling region of tensile member 124 does not twist or otherwise deform as adjustment element 146 is displaced. The present invention contemplates other configurations of housing 144, adjustment element 146, with or without support surfaces 156 and 158, and tensile member 124 suitable to adjustably position lower arch 114 forwardly, relative to upper arch 112, by exerting a tensile force upon lower arch 114.

As shown in FIGURE 9b, tensile member 124 may pass through a hole 162 formed through shaft 148 of adjustment element 146. In one embodiment, adjustment element 146 is a cap screw and hole 162 is formed through an unthreaded portion of shaft 148. As discussed above in connection with FIGURE 9a, adjustment element 146 may be rotated in an appropriate direction to displace adjustment element 146 forwardly, relative to upper arch 112.

When adjustment element 146 is rotated, tensile member 124 contacts and is redirected by first and second hole support surfaces 160 and 164, respectively, located at opposite ends of hole 162. As adjustment element 146 is further rotated, the portions of tensile member 124 located on either side of hole 162 begin to wrap around shaft 148. As tensile member 124 is wrapped around shaft 148 in response to rotation of adjustment element 146, tensile member 124 pulls lower arch 14 forwardly, relative to upper arch 112. The friction forces generated at hole support surfaces 160 and 164 impede or prevent tensile member 124 from moving laterally through hole 162. Therefore, lower arch 114 is displaced equally by first end 126 and second end 128 of tensile member 124 when adjustment element 146 is rotated. The present invention contemplates two or more tensile members coupled to adjustment element 146 using hole 162 or other attachment mechanism in order to replace or combine with the functions of tensile member 124.

As shown in FIGURE 9c, adjustor 134 may include an annular flange 180 that is fixedly coupled to shaft 148 of adjustment element 146. When adjustment element 146 is rotated in an appropriate direction, annular flange 180 is displaced forwardly, relative to upper arch 112, to the same extent as the remainder of adjustment element 146. Annular ring 182 is constructed so as to completely or partially encircle a portion of shaft 148 that is forward of annular flange 180, relative to upper arch 112. Annular ring 182 is coupled to tensile members 123 and 125 using attachment sites 184 and 186, respectively, on annular ring 182. The present invention contemplates any suitable mechanism for coupling tensile members 123 and 125 to annular ring 182. The present invention further contemplates a single tensile member coupled to annular ring 182 to replace of combine with the functions of tensile members 123 and 125.

In one embodiment, shaft 148 of adjustment element 146 is threaded at its distal end, relative to upper arch 112, so as to allow the cap portion of adjustment element 146 to be screwed onto the distal end of adjustment element 146 after annular ring 182 has been seated upon annular flange 180. As a result, annular ring 182 may be constrained to move, if at all, only between the cap portion of adjustment element 46 and annular flange 180. Alternatively, the cap portion of adjustment element 146 may be absent or otherwise removed. In that case, annular ring 182 may be seated upon annular flange 180 and unseated from annular flange 180 free when tensile members 123 and 125 allow.

In one embodiment, annular flange 180 and annular ring 182 operate so as to maintain the rotational orientation of annular ring 182, relative to housing 144, when adjustment element 146 rotates. As a result, tensile members 123 and 125 do not twist or otherwise deform as adjustment element 146 is displaced. When annular flange 180 is displaced forwardly in response to rotation of adjustment element 146, annular ring 182 is displaced forwardly to the same extent, relative to upper arch 112. As annular ring 182 is displaced forwardly, tensile members 123 and 125 are pulled forwardly, relative to upper arch 112. As a result, lower arch 114 is displaced forwardly, relative to upper arch 112, and is displaced equally by tensile member 123 and tensile member 125. The present invention contemplates other configurations of housing 144, adjustment element 146, with or without annular flange 180, and annular ring 182 suitable to adjustably position lower arch 114 forwardly, relative to upper arch 112, by exerting a tensile force upon lower arch 114.

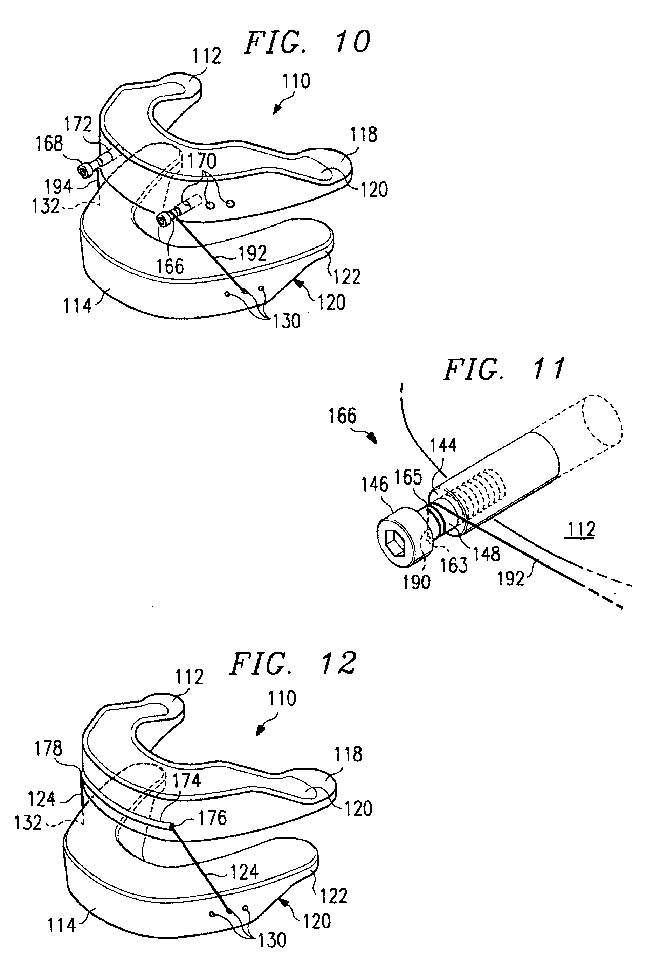

As shown in FIGURE 10, device 110 may include two or more tensile members to replace or combine with the functions of tensile member 124. In one embodiment, tensile members 192 and 194 are coupled to adjustors 166 and 168, respectively, and may be adjusted independently of one another. Adjustors 166 and 168 are coupled to upper arch 112 using attachment sites 170 and 172, respectively. As discussed above in connection with FIGURE 9, the locations of attachment sites 170 and 172 on upper arch 112 are variable. Therefore, adjustors 166 and 168 may be variably positioned according to the particular user or application of device 110. The present invention contemplates adjustors 166 and 168 coupled to lower arch 114 instead of, or in addition to, adjustors 166 and 168 coupled to upper arch 114. For example, adjustors 166 and 168 may be coupled to lower arch 114 using attachment sites 130 and 132, respectively, and tensile members 192 and 194 may be coupled to upper arch 114 using attachment sites 170 and 172, respectively.

Adjustors 166 and 168 may be integral to or separate from upper arch 112. Adjustors 166 and 168 may be formed from the same material as upper arch 112 or from a different material than upper arch 112. As shown in FIGURE 11, adjustors 166 and 168 may be constructed in the same manner as adjustor 134 discussed above in connection with FIGURE 9b. In one embodiment, a stop 163 is coupled to a first end of tensile member 192 and prevents the first end from passing through hole1 90 in shaft 148. The second end of tensile member 192 passes through hole 190 and is coupled to lower arch 114 using a particular attachment site 130.

Similar to the above discussion in connection with FIGURE 9b, when adjustment element 146 is rotated, tensile member 192 contacts and is redirected by a hole support surface 165 located at the end of hole 190 that is opposite stop 163. When adjustment element 146 is rotated, tensile member 192 contacts and is redirected by hole support surface 165. As adjustment element 146 is further rotated, the portion of tensile member 192 located outside of hole 190 begins to wrap around shaft 148. As tensile member 192 is wrapped around shaft 148 in response to rotation of adjustment element 146, tensile member 192 pulls lower arch 114 forwardly, relative to upper arch 112.

Together with stop 163, the friction forces generated between tensile member 192, hole support surface 165, and shaft 148 impede or prevent tensile member 192 from moving within hole 190. Adjustors 166 and 168 are independently adjustable, and the above discussion regarding tensile member 192 and adjustor 166 applies equally to the construction and operation of tensile member 194 and adjustor 168. In combination, tensile members 192 and 194 adjustably position lower arch 114 forwardly, relative to upper arch 112, by exerting a tensile force upon lower arch 114.

As shown in FIGURE 12, device 110 may include a channel 174 to replace or combine with the functions of adjustor 134 and loop support surfaces 136 and 138. In one embodiment, channel 174 receives tensile member 124 along a contact region of tensile member 124 located between first end 126 and second end 128 of tensile member 124. Channel 174 may be integral to or separate from upper arch 112. For example, channel 174 may be formed through a portion of upper arch 112 or channel 174 may be a separate element coupled to upper arch 112 in some manner. Channel 174 may be formed from the same material as upper arch 112 or from a different material than upper arch 112. The present invention contemplates any channel 174 suitable for receiving a portion of tensile member 124.

In one embodiment, channel 174 is formed using a tubular member coupled to the generally forward and downward surface of upper arch 112. Tensile member 124 passes through channel 174, contacts and is redirected by first and second channel support surfaces 176 and 178, respectively, and is coupled to lower arch 114 using attachment sites 130 and 132. As discussed above in connection with FIGURE 8, the length of tensile member 124 and the locations of attachment sites 130 and 132 on lower arch 114 are variable and may be chosen to suit a particular user or application of device 110.

Although friction forces generated by channel support surfaces 176 and 178 may impede or prevent tensile member 124 from moving within channel 174, the present invention contemplates tensile member 124 moving laterally in response to certain movements of the user's lower jaw. The present invention further contemplates a plurality of tensile members to replace or combine with the functions of tensile member 114. Using any suitable combination of adjustor 134, channel 174, and tensile member 124, lower arch 114 may be adjustably positioned forwardly, relative to upper arch 112, by exerting a tensile force upon lower arch 114.

Although the present invention has been described above in connection with several embodiments, it should be understood that a plethora of changes, substitutions, variations, alterations, transformations, and modifications may be suggested to one skilled in the art, and it is intended that the present invention encumbers such changes, variations, substitutions, alterations, transformations, and modifications as fall within the spirit and scope of the appended claims.

## Claims

1. A device (10; 110) for improving the breathing of a user, comprising:
an upper arch (12; 112) adapted to receive at least some of the user's upper teeth;
a lower arch (14; 114) adapted to receive at least some of the user's lower teeth; and
a connector (16; 134) adjustably coupling the upper arch (12) to the lower arch (14);
the connector (16; 134) allowing lateral motion of the lower arch (14; 114) relative to the upper arch (12; 112),
**characterised in that** the connector (16; 134) is operable to be adjusted while the upper arch (12; 112) is coupled to the lower arch (14; 114) and while the device is inserted in the user's mouth to pull the lower arch (14; 114) forwardly to a fixed forward position relative to the upper arch (12; 112).

2. A device (10; 110) as claimed in claim 1, in which the connector (16; 134) is operable to adjustably position the lower arch (14; 114) vertically relative to the upper arch (12; 112).

3. A device (10; 110) as claimed in claim 1, in which the connector (16; 134) is operable to allow the user's lower jaw to move freely in a vertical direction relative to the upper arch (12; 112).

4. A device (10) as claimed in any one of claims 1 to 3, further comprising a hook (44) operable to removably engage the lower arch (14).

5. A device as claimed in claim 4, wherein the hook (44) is adjustably coupled to the connector (16).

6. A device (10) as claimed in claim 4 or claim 5, wherein the hook (44) may be adjusted vertically relative to the upper arch (12).

7. A device (10) as claimed in any one of claims 4 to 6, comprising:
a hooking clasp (34) coupled to the lower arch (14), hook (44) being operable to removably engage the hooking clasp (34).

8. A device (10) as claimed in any one of claims 1 to 3, in which the connector (16) comprises a flexible member (46) coupling the lower arch (14) to the connector (16).

9. A device (10) as claimed in any one of claims 1 to 8, in which the connector (16) is operable to constrain within a desired range the vertical movement of the lower arch (14) relative to the upper arch (12).

10. A device (10) as claimed in any one of claims 1 to 9, in which the connector (16) comprises a slider (96) and a connecting arm (94), the connecting arm (94) coupling to the upper arch (12), the slider (96) operable to be adjusted to pull the lower arch (14) forwardly.

11. A device (10) as claimed in any one of claims 1 to 10, further comprising a face mask (86) adjustably coupled to the connector(16).

12. A device as claimed in claim 11, and further comprising:
a connecting apparatus (67) adjustably coupling the connector (16) to the face mask (86), the connecting apparatus (67) comprising:
a plurality of swivel collars (70);
a first post (84) coupled to the face mask (86) ;
a second post (68) coupled to the upper arch (12) ;
at least one connecting post (76), the first post (84) being adjustably coupled to the second post (68) through a series of collar joints, each of the collar joints being formed by the coupling of one of the plurality of swivel collars (70) with at least a selected one of the first (84), second (68), and connecting posts (76).

13. A device (10; 110) as claimed in any one of claims 1 to 3, in which the connector (16; 134) comprises a tensile member (48, 124) that may be adjusted to pull the lower arch (14; 114) forwardly.

14. A device as claimed in claim 13, in which the tensile member (124) is coupled to the lower arch (114) at ends (126, 128) of the tensile member (124) the tensile member (124) coupled to the upper arch (112) between the ends (126, 128).

15. A device (110) as claimed in claim 13 or claim 14, further comprising:
a housing (144) coupled to the upper arch (12);
an adjustment element (146) coupled to the tensile member (124) and the housing (144), the adjustment element (146) operable to pull the lower arch (114) forward when the adjustment element (146) is displaced forwardly relative to the housing (144).

16. A device (110) as claimed in any one of claims 13 to 15, in which the tensile member (124) has first and second ends (126, 128) coupled to the lower arch (114) at first and second attachment sites (130, 132), respectively, the locations of the first and second attachment sites (130, 132) being variable.

17. A device (110) as claimed in any one of claims 13 to 16, further comprising a channel (174) operable to slidingly receive the tensile member (124).

## Patentansprüche

1. Vorrichtung (10; 110) zur Verbesserung der Atmung eines Benutzers, die umfasst:
einen oberen Bogen (12; 112), der dafür ausgelegt ist, mindestens einige der oberen Zähne des Benutzers aufzunehmen;
einen unteren Bogen (14; 114), der dafür ausgelegt ist, mindestens einige der unteren Zähne des Benutzers aufzunehmen; und
ein Verbindungsstück (16; 134), das den oberen Bogen (12) mit dem unteren Bogen (14) einstellbar verbindet;
wobei das Verbindungsstück (16; 134) eine seitliche Bewegung des unteren Bogens (14; 114) im Verhältnis zum oberen Bogen (12; 112) ermöglicht;
**dadurch gekennzeichnet, dass** das Verbindungsstück (16; 134) eingestellt werden kann, während der obere Bogen (12; 112) mit dem unteren Bogen (14; 114) verbunden ist und die Vorrichtung im Mund eines Benutzers eingeführt ist, um den unteren Bogen (14; 114) im Verhältnis zum oberen Bogen (12; 112) nach vorn in eine feste Vorwärtsposition zu ziehen.

2. Vorrichtung (10; 110) nach Anspruch 1, wobei das Verbindungsstück (16; 134) den unteren Bogen (14; 114) vertikal im Verhältnis zum oberen Bogen (12; 112) einstellbar positionieren kann.

3. Vorrichtung (10; 110) nach Anspruch 1, wobei das Verbindungsstück (16; 134) die freie Bewegung des Unterkiefers des Benutzers in vertikaler Richtung im Verhältnis zum oberen Bogen (12; 112) ermöglicht.

4. Vorrichtung (10) nach einem der Ansprüche 1 bis 3, die weiterhin einen Haken (44) umfasst, der entfernbar in den unteren Bogen (14) eingreift.

5. Vorrichtung nach Anspruch 4, wobei der Haken (44) mit dem Verbindungsstück (16) einstellbar verbunden ist.

6. Vorrichtung (10) nach Anspruch 4 oder 5, wobei der Haken (44) im Verhältnis zum oberen Bogen (12) vertikal eingestellt werden kann.

7. Vorrichtung (10) nach einem der Ansprüche 4 bis 6, die Folgendes umfasst:
eine Einhakspange (34), die mit dem unteren Bogen (14) verbunden ist, wobei der Haken (44) entfernbar in die Einhakspange (34) eingreift.

8. Vorrichtung (10) nach einem der Ansprüche 1 bis 3, wobei das Verbindungsstück (16) ein elastisches Element (46) umfasst, das den unteren Bogen (14) mit dem Verbindungsstück (16) verbindet.

9. Vorrichtung (10) nach einem der Ansprüche 1 bis 8, wobei das Verbindungsstück (16) die vertikale Bewegung des unteren Bogens (14) im Verhältnis zum oberen Bogen (12) innerhalb eines gewünschten Bereichs einschränkt.

10. Vorrichtung (10) nach einem der Ansprüche 1 bis 9, wobei das Verbindungsstück (16) eine Führungsstange (96) und einen Verbindungsarm (94) umfasst, der Verbindungsarm (94) mit dem oberen Bogen (12) verbunden ist und die Führungsstange (96) so eingestellt werden kann, dass sie den unteren Bogen (14) nach vorn zieht.

11. Vorrichtung (10) nach einem der Ansprüche 1 bis 10, die weiterhin eine Gesichtsmaske (86) umfasst, die mit dem Verbindungsstück (16) einstellbar verbunden ist.

12. Vorrichtung nach Anspruch 11, die weiterhin Folgendes umfasst:
eine Verbindungseinrichtung (67), die das Verbindungsstück (16) einstellbar mit der Gesichtsmaske (86) verbindet, wobei die Verbindungseinrichtung (67) Folgendes umfasst:
eine Vielzahl von Drehzwingen (70);
eine erste Stange (84), die mit der Gesichtsmaske (86) verbunden ist;
eine zweite Stange (68), die mit dem oberen Bogen (12) verbunden ist;
mindestens eine Verbindungsstange (76), wobei die erste Stange (84) mit der zweiten Stange (68) mit Hilfe einer Reihe von Zwingenverbindungen einstellbar verbunden ist und jede der Zwingenverbindungen gebildet wird, indem eine der Vielzahl der Drehzwingen (70) mit mindestens einer Stange, die aus der ersten Stange (84), der zweiten Stange (68) oder der Verbindungsstange (76) ausgewählt wird, verbunden wird.

13. Vorrichtung (10; 110) nach einem der Ansprüche 1 bis 3, wobei das Verbindungsstück (16; 134) ein Dehnelement (48, 124) umfasst, das so eingestellt werden kann, dass der untere Bogen (14; 114) nach vorn gezogen wird.

14. Vorrichtung nach Anspruch 13, wobei das Dehnelement (124) an den Enden (126, 128) des Dehnelements (124) mit dem unteren Bogen (114) verbunden ist und das Dehnelement (124) zwischen den Enden (126, 128) mit dem oberen Bogen (112) verbunden ist.

15. Vorrichtung (110) nach Anspruch 13 oder 14, die weiterhin Folgendes umfasst:
ein Gehäuse (144), das mit dem oberen Bogen (12) verbunden ist;
ein Einstellelement (146), das mit dem Dehnelement (124) und dem Gehäuse (144) verbunden ist, wobei das Einstellelement (146) den unteren Bogen (114) nach vorn zieht, wenn das Einstellelement (146) im Verhältnis zum Gehäuse (144) nach vorn verschoben wird.

16. Vorrichtung (110) nach einem der Ansprüche 13 bis 15, wobei das Dehnelement (124) ein erstes und zweites Ende (126, 128) aufweist, die mit dem unteren Bogen (114) jeweils an einer ersten und zweiten Befestigungsstelle (130, 132) verbunden sind, und wobei die Positionen der ersten und zweiten Befestigungsstelle (130, 132) variabel sind.

17. Vorrichtung (110) nach einem der Ansprüche 13 bis 16, die weiterhin einen Kanal (174) umfasst, der das Dehnelement (124) gleitend aufnimmt.

## Revendications

1. Dispositif (10 ; 110) pour améliorer la respiration d'un utilisateur, comprenant :
une arche supérieure (12 ; 112) adaptée pour recevoir au moins certaines des dents supérieures de l'utilisateur ;
une arche inférieure (14 ; 114) adaptée pour recevoir au moins certaines des dents inférieures de l'utilisateur ; et
un connecteur (16 ; 134) couplant de manière réglable l'arche supérieure (12) à l'arche inférieure (14) ;
le connecteur (16 ; 134) permettant un mouvement latéral de l'arche inférieure (14 ; 114) par rapport à l'arche supérieure (12; 112) ;
**caractérisé en ce que** le connecteur (16 ; 134) est actionnable pour être réglé pendant que l'arche supérieure (12 ; 112) est couplée à l'arche inférieure (14 ; 114) et pendant que le dispositif est inséré dans la bouche de l'utilisateur afin de tirer l'arche inférieure (14 ; 114) vers l'avant jusqu'à une position avant fixe par rapport à l'arche supérieure (12 ; 112).

2. Dispositif (10 ; 110) selon la revendication 1, dans lequel le connecteur (16 ; 134) est actionnable pour positionner de manière réglable l'arche inférieure (14 ; 114) verticalement par rapport à l'arche supérieure (12 ; 122).

3. Dispositif (10 ; 110) selon la revendication 1, dans lequel le connecteur (16 ; 134) est actionnable pour permettre à la mâchoire inférieure de l'utilisateur de se déplacer librement dans un sens vertical par rapport à l'arche supérieure (12 ; 112).

4. Dispositif (10) selon l'une quelconque des revendications 1 à 3, comprenant en outre un crochet (44) actionnable pour engager de manière détachable l'arche inférieure (14).

5. Dispositif selon la revendication 4, dans lequel le crochet (44) est couplé de manière réglable au connecteur (16).

6. Dispositif (10) selon la revendication 4 ou la revendication 5, dans lequel le crochet (44) peut être réglé verticalement par rapport à l'arche supérieure (12).

7. Dispositif (10) selon l'une quelconque des revendications 4 à 6, comprenant :
un fermoir d'accrochage (34) couplé à l'arche inférieure (14), le crochet (44) étant actionnable pour engager de manière détachable le fermoir d'accrochage (34).

8. Dispositif (10) selon l'une quelconque des revendications 1 à 3, dans lequel le connecteur (16) comprend un élément souple (46) couplant l'arche inférieure (14) au connecteur (16).

9. Dispositif (10) selon l'une quelconque des revendications 1 à 8, dans lequel le connecteur (16) est actionnable pour contraindre dans une gamme désirée le mouvement vertical de l'arche inférieure (14) par rapport à l'arche supérieure (12).

10. Dispositif (10) selon l'une quelconque des revendications 1 à 9, dans lequel le connecteur (16) comprend un curseur (96) et un bras de connexion (94), le bras de connexion (94) se couplant à l'arche supérieure (12), le curseur (96) étant actionnable pour être réglé afin de tirer l'arche inférieure (14) vers l'avant.

11. Dispositif (10) selon l'une quelconque des revendications 1 à 10, comprenant en outre un masque facial (86) couplé de manière réglable au connecteur (16).

12. Dispositif selon la revendication 11, et comprenant en outre :
un dispositif de connexion (67) couplant de manière réglable le connecteur (16) au masque facial (86), le dispositif de connexion (67) comprenant :
une pluralité de colliers articulés (70) ;
un premier montant (84) couplé au masque facial (86) ;
un deuxième montant (68) couplé à l'arche supérieure (12) ;
au moins un montant de connexion (76), le premier montant (84) étant couplé de manière réglable au deuxième montant (68) par le biais d'une série de joints de collier, chacun des joints de collier étant formé par le couplage d'un de la pluralité de colliers articulés (70) avec au moins un montant sélectionné du premier montant (84), du deuxième montant (68) et des montants de connexion (76).

13. Dispositif (10 ; 110) selon l'une quelconque des revendications 1 à 3, dans lequel le connecteur (16 ; 134) comprend un élément de traction (48, 124) qui peut être réglé pour tirer l'arche inférieure (14 ; 114) vers l'avant.

14. Dispositif selon la revendication 13, dans lequel l'élément de traction (124) est couplé à l'arche inférieure (114) au niveau d'extrémités (126, 128) de l'élément de traction (124), l'élément de traction (124) étant couplé à l'arche supérieure (112) entre les extrémités (126, 128).

15. Dispositif (110) selon la revendication 13 ou la revendication 14, comprenant en outre :
un logement (144) couplé à l'arche supérieure (12) ;
un élément de réglage (146) couplé à l'élément de traction (124) et au logement (144), l'élément de réglage (146) étant actionnable pour tirer l'arche inférieure (114) vers l'avant quand l'élément de réglage (146) est déplacé vers l'avant par rapport au logement (144).

16. Dispositif (110) selon l'une quelconque des revendications 13 à 15, dans lequel l'élément de traction (124) comporte des première et deuxième extrémités (126, 128) couplées à l'arche inférieure (114) au niveau de premier et deuxième sites de fixation (130, 132), respectivement, les emplacements des premier et deuxième sites de fixation (130, 132) étant variables.

17. Dispositif (110) selon l'une quelconque des revendications 13 à 16, comprenant en outre une rainure (174) actionnable pour recevoir de manière coulissante l'élément de traction (124).
